Europäisches Patentamt

**European Patent Office**

**Office européen des brevets**

(19)

(11) Numéro de publication: **0 046 430**
A1

## (12) DEMANDE DE BREVET EUROPEEN

(21) Numéro de dépôt: **81401277.9**

(22) Date de dépôt: **07.08.81**

(51) Int. Cl.³: **G 01 N 21/25**, G 01 N 33/48

(30) Priorité: **14.08.80 FR 8017966**

(43) Date de publication de la demande: **24.02.82**
Bulletin 82/8

(84) Etats contractants désignés: **BE CH DE FR GB IT LI NL**

(71) Demandeur: **COMMISSARIAT A L'ENERGIE ATOMIQUE Etablissement de Caractère Scientifique Technique et Industriel, 31/33, rue de la Fédération, F-75015 Paris (FR)**
Demandeur: **LE MATERIEL BIOMEDICAL Société à Responsabilité Limitée dite:, 4 rue de Presbourg, F-75116 Paris (FR)**

(72) Inventeur: **Roche, Pierre, 155 av. de la République, F-94700 Maisons-Alfort (FR)**
Inventeur: **Marcelli, Aline, 24 avenue Duquesne, F-75007 Paris (FR)**

(74) Mandataire: **Bonnetat, Christian et al, Cabinet PROPI Conseils 23 rue de Léningrad, F-75008 Paris (FR)**

(54) **Procédé et dispositif pour la détection et la quantification d'agglutinats en temps réel.**

(57) Procédé et dispositif pour la détection et la quantification d'agglutinats en temps réel.

Selon l'invention, au moyen d'une pluralité de dispositifs photosensibles, on effectue dans des conditions comparables à celles de l'observation correspondant à la détection des éventuels agglutinats, au moins une observation auxiliaire du fond (11) du récipient (10) ou d'un récipient semblable, le récipient (10) ainsi observé étant vide ou contenant un liquide transparent et on corrige l'observation correspondant à la détection des éventuels agglutinats en fonction de cette observation auxiliaire, de façon à rendre homogènes les réponses individuelles des différents dispositifs photosensibles.

## Procédé et dispositif pour la détection et la quantification d'agglutinats en temps réel.

La présente invention concerne un procédé et un dispositif pour la détection et la quantification, dans un récipient dont au moins le fond est transparent, d'agglutinats susceptibles d'être formés, sous l'action d'au moins un réactif, par des particules en suspension dans un liquide se trouvant dans ledit récipient, le fond dudit récipient contenant les éventuels agglutinats étant observé par transparence au moyen d'un agencement comportant une pluralité de dispositifs photosensibles balayant le fond dudit récipient, de façon à former n x m points d'observation répartis selon une surface rectangulaire ou carrée occupant la plus grande partie de la surface dudit fond.

L'invention peut être utilisée chaque fois que l'on a à détecter et à quantifier des agglutinats. Cependant, elle s'applique particulièrement bien en immunohématologie, notamment en vue de la détermination des groupes sanguins. On sait en effet, que la détermination des groupes sanguins est liée à la recherche de l'existence dans le sang d'antigènes érythrocytaires. Les réactions immunocytologiques, face à un antigène de groupe spécifique, se traduisent par un phénomène d'agglutination des hématies, si ces dernières appartiennent à un autre groupe. Ces hématies peuvent former soit des agglutinats uniformes sans hématies libres, soit quelques gros agglutinats, ou bien encore un grand nombre de petits agglutinats.

On connaît déjà par la demande de brevet français 2 444 939 un dispositif du type décrit ci-dessus.

0046430

Un tel dispositif est particulièrement avantageux, puisqu'il permet d'utiliser des détecteurs formés par un circuit à couplage de charges et donc de profiter de l'excellente analyse d'images procurée par un tel circuit. Cependant, le fonctionnement pratique d'un tel dispositif soulève des difficultés. En effet, les réponses des différentes diodes à couplage de charges individuelles ne sont pas homogènes, ce qui fausse les résultats obtenus.

La présente invention a pour objet de remédier à cet inconvénient et elle permet de lire et de quantifier automatiquement, avec précision et en temps réel, les agglutinats d'hématies.

A cette fin, selon l'invention, le procédé du type mentionné ci-dessus est caractérisé en ce que l'on effectue, dans des conditions comparables à celles de l'observation correspondant à la détection des éventuels agglutinats, au moins une observation auxiliaire du fond dudit récipient ou d'un récipient semblable, le récipient ainsi observé étant vide ou contenant un liquide transparent et on corrige l'observation correspondant à la détection des éventuels agglutinats en fonction de cette observation auxiliaire, de façon à rendre homogènes les réponses individuelles des différents dispositifs photosensibles.

On remarquera que le brevet britannique 2 014 300 décrit déjà une telle observation auxiliaire. Cependant, dans ce cas, le dispositif d'observation ne comporte qu'un seul détecteur individuel par cellule, de sorte que l'observation auxiliaire n'est pas destinée à homogénéiser les réponses d'une multiplicité de dispositifs photosensibles d'observation, mais uniquement à prendre en compte dans la mesure, le fond dudit récipient.

De préférence, dans la mise en oeuvre du procédé conforme à l'invention, on observe, dans des conditions comparables à celles de l'observation correspondant à la détection des éventuels agglutinats et dans le même récipient ou dans un récipient semblable, un échantillon témoin du liquide dans lequel lesdites particules ne sont pas agglutinées, on corrige cette observation de l'échantillon témoin en fonction de l'observation auxiliaire mentionnée ci-dessus, et on compare les indications obtenues dans les deux observations corrigées pour déterminer, dans la première citée, l'existence et l'importance d'une éventuelle agglutination.

Dans un mode avantageux de réalisation, l'agencement de dispositifs photosensibles est formé de n dispositifs photosensibles alignés et le balayage du fond du récipient, lors de l'observation des éventuels agglutinats, est obtenue par m déplacements relatifs dudit agencement par rapport au fond, tandis que l'observation auxiliaire et l'observation de l'échantillon témoin sont effectuées dans une seule position relative de l'agencement par rapport au fond du récipient, correspondant au moins approximativement au centrage dudit agencement par rapport à ce dernier. Bien entendu, cette mesure avantageuse n'est pas limitative et l'observation auxiliaire et l'observation de l'échantillon témoin pourraient être effectuées dans des conditions de balayage identiques à celles de l'observation des éventuels agglutinats.

Les dispositifs photosensibles sont avantageusement du type à couplage ou à transfert de charge, ces dispositifs étant généralement désignés par les lettres CCD, DTC ou CCPD.

Dans le balayage du fond du récipient, lors de l'observation des éventuels agglutinats, on peut bien entendu choisir n = m et faire en sorte que l'écart entre deux positions d'observation consécutives de la barrette soit égal à l'écart entre deux dispositifs CCD de la barrette. Ainsi, on couvre totalement une surface carrée du fond transparent. Par exemple, la barrette rectiligne comporte 256 diodes CCD, ayant chacune une définition de 25 µm, l'information vidéo étant numérisée en 256 niveaux de gris, et l'image analysée représente un carré centré sur ledit fond transparent et formé par 256 balayages écartés de 25 µm.

On obtient ainsi, pour l'observation des agglutinats, n x m mesures, réparties en m balayages de n points.

Il est avantageux, lors de l'observation de l'échantillon témoin et après correction par l'observation auxiliaire, d'effectuer la moyenne des réponses des dispositifs photosensibles de l'agencement et d'utiliser cette moyenne pour établir au moins un seuil moyen de référence.

De préférence, à partir de cette moyenne, on établit un seuil minimum et un seuil maximum, respectivement inférieur et supérieur à cette moyenne de quantités choisies volontairement en fonction de l'analyse effectuée et des connaissances que l'on en a, puis, lors de l'observation des éventuels agglutinats, on compare les réponses des dispositifs photosensibles de l'agencement pour chacun des n x m points d'observation audit seuil minimum et maximum et l'on répartit lesdites réponses en trois classes différentes, selon qu'elles sont supérieures au seuil maximum, inférieures au seuil minimum
ou comprises entre ces deux seuils.

Un dispositif selon l'invention, pour mettre en oeuvre
le procédé décrit ci-dessus, comporte avantageusement,
à poste fixe, d'une part une source de lumière et
d'autre part un système optique et un agencement de
dispositifs photosensibles associés à des moyens de
mémorisation des signaux qui en sont issus, des moyens
mobiles de support pour faire défiler ledit récipient
entre ladite source de lumière et ledit système optique,
des moyens de commande desdits dispositifs photosensibles, des moyens moteurs pour mouvoir lesdits moyens
mobiles de support, des moyens de commande desdits
moyens moteurs, des moyens de synchronisation entre
lesdits moyens de commande des dispositifs photosensibles et lesdits moyens de commande des moyens moteurs
et des moyens de traitement des signaux provenant desdits
moyens de mémorisation.

Les moyens de commande des dispositifs photosensibles , les moyens de commande des moyens moteurs,
lesdits moyens de synchronisation et lesdits moyens
de traitement peuvent être rassemblés dans un microprocesseur.

Dans le cas d'une pluralité d'alvéoles solidaires d'une
plaque transparente, lesdits moyens moteurs sont constitués de deux moteurs pas à pas croisés, tandis que
lesdits moyens mobiles de support sont constitués de
deux tables mobiles croisées, dont chacune d'elles est
mue par l'un desdits moteurs.

Les figures du dessin annexé feront bien comprendre comment l'invention peut être réalisée.

La figure 1 est une vue schématique en perspective du dispositif selon l'invention.

La figure 2 est une vue en plan d'une plaquette à alvéoles utilisée en combinaison avec le dispositif de la figure 1.

La figure 3 est une coupe suivant la ligne III-III de la figure 2.

Les figures 4,5 et 6 illustrent schématiquement une partie du fonctionnement du dispositif selon l'invention.

Les figures 7 et 8 sont des courbes de résultat de tests effectués au moyen du dispositif selon l'invention.

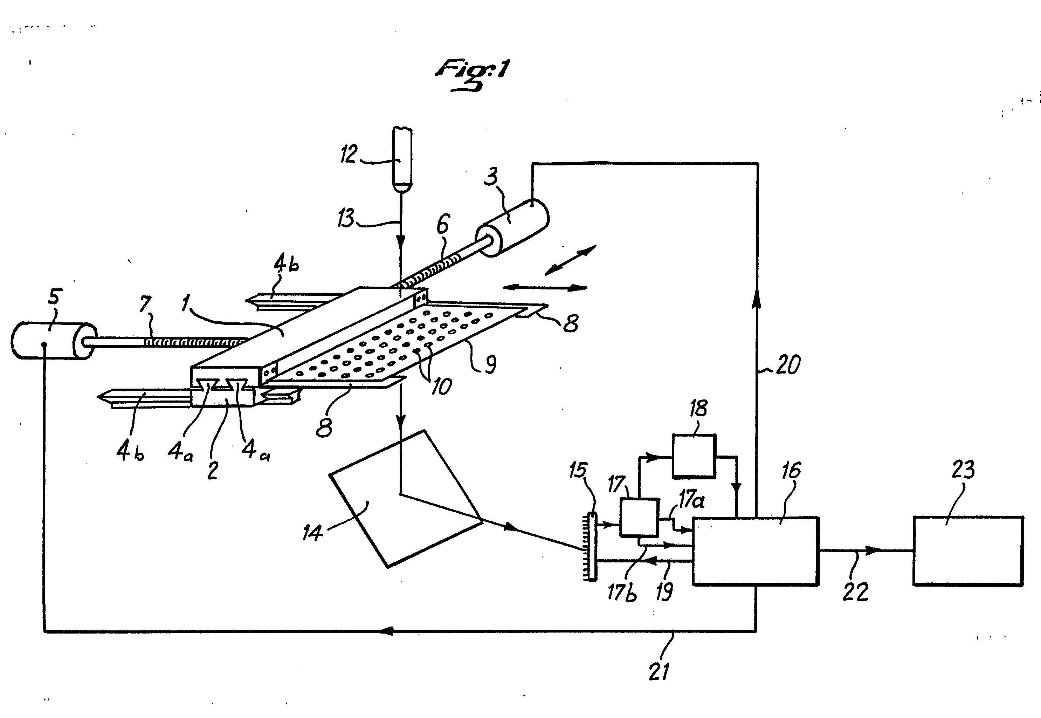

Le dispositif selon l'invention montré par la figure 1, comporte deux tables 1 et 2 à coulissement croisé. La table 1 peut glisser, sous l'action d'un moteur pas-à-pas 3, le long des rails 4a solidaires de la table 2. De façon semblable, la table 2 peut glisser sous l'action d'un moteur 5, le long de rails 4b solidaires du bâti non représenté du dispositif. Les rails 4a et 4b sont orthogonaux. La liaison entre le moteur 3 et la table 1 d'une part, et entre le moteur 5 et la table 2 d'autre part, est réalisée au moyen de vis sans fin 6 et 7 engagées dans des écrous (non-visibles sur la figure) respectivement solidaires desdites tables 1 et 2.

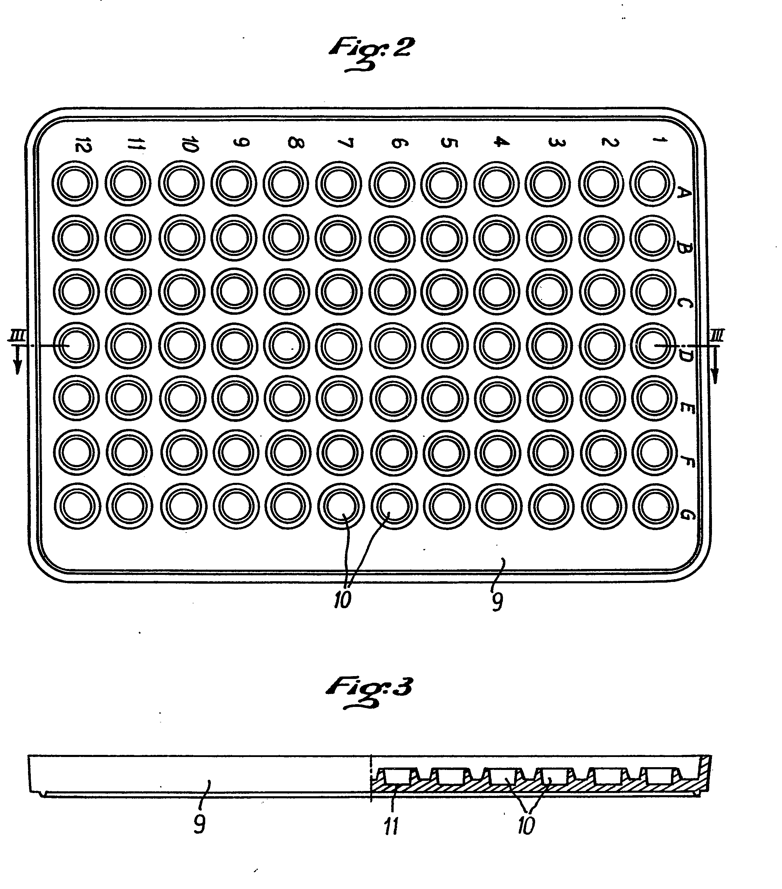

Des bras saillants 8, solidaires de la table 1, sont prévus pour supporter, en porte-à-faux, une plaquette transparente 9, pourvue d'une pluralité d'alvéoles 10.
Comme le montre les figures 2 et 3, les alvéoles 10 sont
répartis en sept colonnes, référencées de A à G, de
douze alvéoles chacune. De façon connue la plaque 9
est moulée dans une matière transparente, chacun desdits alvéoles étant ouvert vers le haut et ayant une
forme conique ou cylindrique, avec un fond 11 plat.
Au point de vue optique chaque fond 11 forme une lame
plane à faces parallèles.

Au-dessus de la plaque 9 est prévue une source lumineuse 12 constante pendant la durée de l'analyse, par
exemple à faisceau de fibres optiques, émettant un
faisceau lumineux 13. Au dessous de la plaque 9 est
disposé un miroir réfléchissant 14 dirigeant le faisceau
13, après une traversée de la plaque 9, sur une barrette
15 comportant une pluralité de dispositifs à couplage
de charges alignées.

Une unité de commande, de traitement et de calcul 16
reçoit, par un aiguilleur 17 et une mémoire 18, les
informations recueillies par la barrette 15 et, par des
liaisons 19,20, 21 et 22, commande respectivement
ladite barrette 15, le moteur 5 et un dispositif d'affichage 23.

On conçoit aisément que par coulissement croisé des
tables 1 et 2 dans des directions et sens indiqués par
les flèches de la figure 1 et parallèles aux rails 4a
et 4b, il est possible d'amener successivement chacun
des alvéoles 10 de la plaque 9 dans le faisceau 13,
c'est-à-dire dans le champ de la barrette 15. Par exemple, c'est d'abord l'alvéole 1 de la colonne A qui se
trouve en regard de la source 12, puis l'alvéole 2 de
la colonne A, etc... jusqu'à l'alvéole 12 de la

8 0046430

colonne A, après quoi l'alvéole 1 de la colonne B est amené dans le faisceau 13, puis l'alvéole 2 de la colonne B, etc... jusqu'à l'alvéole 12 de la colonne G.

Les douze alvéoles de chacune des colonnes A à G peuvent être destinés aux tests concernant chacun une personne, de sorte que des tests concernant sept personnes peuvent être effectués à l'aide d'une seule plaquette 9.

Par exemple, dans chacune des colonnes d'alvéoles A à G, le premier alvéole est vide (ou rempli de sérum physiologique) et le second alvéole contient un tapis d'hématies libres, alors que les dix autres alvéoles suivants contiennent du sang additionné de divers réactifs susceptibles d'entrainer des agglutinations d'hématies.

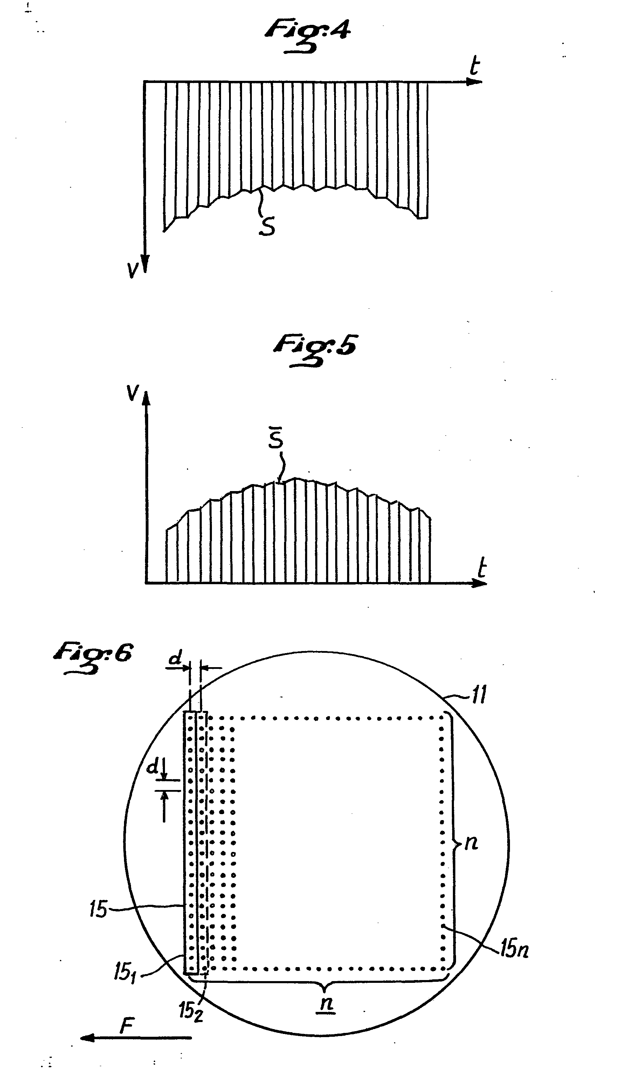

A partir d'une position initiale définie grâce à des capteurs de positions (non représentés), la plaquette 10 est d'abord amenée dans une position pour laquelle le fond du premier alvéole d'une colonne (vide ou rempli de sérum physiologique) est projetée sur la barrette 15, celle-ci étant centrée par rapport audit fond. On effectue alors une mesure, de sorte que l'on obtient pour un écartement constant, un signal S irrégulier (voir la figure 4). L'irrégularité du signal S est due à l'inhomogénéité des diodes CCD de la barrette 15 et aux déformations optiques du fond 11. Sur la figure 4, le signal S est représenté par un niveau de tension V en fonction du temps $t$, c'est-à-dire en réalité de la succession des 256 diodes de la barrette 15. Le signal S est transmis, par l'aiguilleur 17, à la mémoire 18, de type FIFO (first in, first out), à laquelle est associé un inverseur (non-représenté), de sorte qu'à la sortie de ladite mémoire apparait le signal $\overline{S}$ de la figure 5.

Ensuite, sous l'action des moteurs 3 et 5 et sous la commande de l'unité 16, la plaquette 9 est déplacée de façon que le fond du second alvéole de la même colonne (contenant un tapis d'hématies libres) soit projeté sur la barrette 15, en position centrée. Une mesure à cet instant fournit un signal témoin constitué par les réponses des 256 diodes. Ce signal témoin est transmis, par l'aiguilleur 17 et la liaison 17a, à l'unité centrale 16 où il est additionné au signal $\overline{S}$. Il est donc corrigé des déformations optiques et des inhomogénéités de réponse des diodes. Les 256 réponses ainsi corrigées des diodes sont additionnées et divisées par 256 par ladite unité 16 de sorte que l'on obtient une moyenne M servant de seuil moyen de référence. A partir de cette moyenne M, on détermine un seuil minimum $S_{min} = M - \varepsilon$ et un seuil maximum $S_{max} = M + \varepsilon$, $\varepsilon$ étant choisi volontairement en fonction de l'analyse effectuée et des connaissances que l'on en a.

Ensuite, on effectue la mesure de chacun des alvéoles suivants de la même colonne. Lorsqu'un de ces dix alvéoles susceptible de contenir des agglutinats est amené dans le champ de la barrette 15, on effectue une pluralité de mesures, selon le processus illustré par la figure 6. Sur cette figure, on a supposé que l'image du fond 11 d'un alvéole 10 est superposé à la barrette 15, que ledit fond défilait de la droite vers la gauche, selon la flèche F et que la barrette 15 comprenait $n$ diodes CCD, équidistantes de la distance $d$. L'unité 16 commande les moteurs 3 et 5 pour que la barrette 15 prenne une position $15_1$ initiale extrême et une mesure est effectuée de sorte que les $n$ diodes CCD fournissent leur éclairement reçu dans cette position.

La mesure est transmise, par l'aiguilleur 17 et la liaison 17b, à l'unité 16 qui la linéarise grâce au signal $\bar{S}$ provenant de la mémoire 18. On obtient alors une valeur $N_1$ par diode

Si $N_1 > S_{max}$ , on peut en déduire qu'il y a agglutination.

Si $N_1 < S_{min}$ , $N_1$ correspond au fond de l'image. et,

Si $S_{min} < N_1 > S_{max}$ , il n'y a pas agglutination.

L'unité 16 comporte trois compteurs (non-représentés), dont chacun correspond à l'une des trois classes précédentes et vers lesquelles sont dirigées les valeurs $N_1$ obtenues qui sont stockées dans le compteur approprié.

Ensuite, la barrette 15 est amenée dans la position $15_2$ voisine, parallèle et distante de $\underline{d}$, où une mesure est de nouveau effectuée. On obtient de nouvelles valeurs $N_2$, qui sont également comparées à $S_{max}$ et $S_{min}$ et stockées dans le compteur correspondant. On continue ainsi, jusqu'à ce que l'on arrive dans la position finale extrême $15_n$, où les dernières valeurs $N_n$ sont classées et emmagasinées.

Des contenus des trois compteurs, l'unité 16 déduit s'il y a agglutination ou non et détermine le type de l'éventuelle agglutination. Le résultat est affiché en 23.

Ensuite, l'unité 16 commande les moteurs 3 et 5 pour amener l'alvéole suivant dans le champ de la barrette 15, etc...

La figure 6 montre que chaque alvéole susceptible de contenir des agglutinats subit une observation en $n^2$ points formant un carré inscrit dans le fond 11.

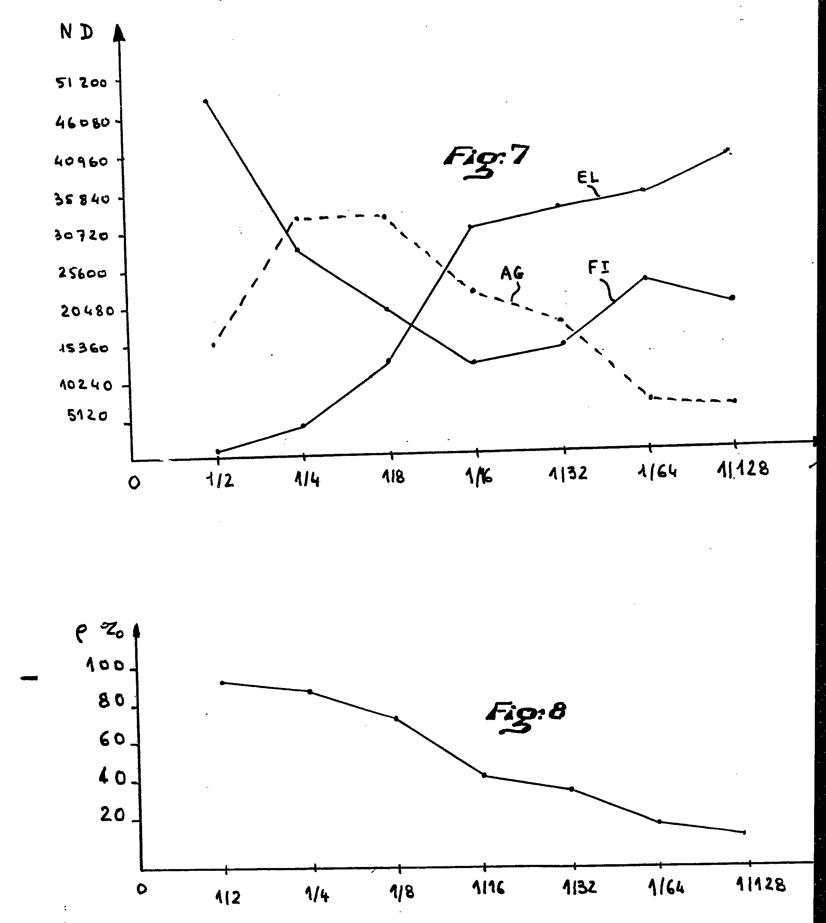

Les figures 7 et 8 illustrent le résultat de tests réalisés, sur une suspension d'hématies de groupe A au moyen d'un sérum contenant un anti-corps anti-A. La figure 7 représente, en fonction de la dilution $\underline{c}$ du sérum (de $\frac{1}{2}$ à $\frac{1}{128}$ ) dans de l'eau physiologique, le nombre ND de diodes CCD du dispositif 15, occultées pendant le processus de balayage illustré par la figure 6, respectivement par les agglutinats (courbe AG) , les érythrocytes libres (courbe EL) et le fond de l'image (courbe FI).

Sur la figure 8, on a porté (en %) le rapport

$$\rho = \frac{\text{Nombre de diodes CCD occultées par les agglutinats}}{\begin{array}{l}\text{Nombre de diodes CCD occultées par les agglutinats}\\ \text{+ Nombre de diodes CCD correspondant aux érythro-}\\ \text{cytes libres}\end{array}}$$

en fonction de la dilution $\underline{c}$.

Ces courbes montrent que, pour une suspension d'hématies donnée, le rapport $\rho$ varie de façon quantifiée en fonction de la dilution et que, en conséquence, le procédé et le dispositif selon l'invention permettent la quantification des agglutinats. De plus, comme il a été expliqué ci-dessus, cette quantification s'effectue en temps réel.

REVENDICATIONS

1 - Procédé pour la détection et la quantification, dans un récipient dont au moins le fond est transparent, d'agglutinats susceptibles d'être formés, sous l'action d'au moins un réactif, par des particules en suspension dans un liquide se trouvant dans ledit récipient, selon lequel on observe par transparence le fond (11) dudit récipient (10) contenant les éventuels agglutinats au moyen d'un agencement (15) comportant une pluralité de dispositifs photosensibles balayant le fond (11) du récipient (10), de façon à former n x m points d'observation répartis selon une surface rectangulaire ou carrée occupant la plus grande partie de la surface dudit fond (11), caractérisé en ce que l'on effectue dans des conditions comparables à celles de l'observation correspondant à la détection des éventuels agglutinats, au moins une observation auxiliaire du fond (11) dudit récipient (10) ou d'un récipient semblable, le récipient (10) ainsi observé étant vide ou contenant un liquide transparent et on corrige l'observation correspondant à la détection des éventuels agglutinats en fonction de cette observation auxiliaire, de façon à rendre homogènes les réponses individuelles des différents dispositifs photosensibles.

2 - Procédé selon la revendication 1, caractérisé en ce que l'on observe, dans des conditions comparables à celles de l'observation correspondant à la détection des éventuels agglutinats et dans le même récipient (10) ou dans un récipient semblable, un échantillon témoin dudit liquide dans lequel lesdites particules ne sont pas agglutinées, en ce que l'on corrige cette observation de l'échantillon témoin en fonction de l'observation auxiliaire, et en ce que l'on compare les indications obtenues dans les deux observations corrigées pour déterminer, dans la première citée, l'existence et l'importance d'une éventuelle agglutination.

3.- Procédé selon l'une quelconque des revendications 1 ou 2, caractérisé en ce que l'agencement (15) est formé de $n$ dispositifs photosensibles alignés et en ce que le balayage du fond (11) du récipient (10) lors de l'observation des éventuels agglutinats, est obtenu par $m$ déplacements relatifs dudit agencement (15) par rapport audit fond (11).

4.- Procédé selon l'une quelconque des revendications 1 à 3, caractérisé en ce que les dispositifs photosensibles sont du type à couplage ou à transfert de charges.

5.- Procédé selon l'une quelconque des revendications 1 à 4, caractérisé en ce que, lors de l'observation de l'échantillon témoin et après correction par l'observation auxiliaire, on effectue la moyenne des réponses des dispositifs photosensibles de l'agencement (15) et on utilise cette moyenne pour établir au moins un seuil moyen de référence.

0046430

6 - Procédé selon la revendication 5,
caractérisé en ce que, à partir de ladite moyenne, on
établit un seuil minimum et un seuil maximum respectivement inférieur et supérieur à cette moyenne de quantités choisies volontairement en fonction de l'analyse
effectuée et des connaissances que l'on en a, en ce
que, lors de l'observation des éventuels agglutinats,
on compare la réponse des dispositifs photosensibles
de l'agencement (15) pour chacun des n x m points
d'observation auxdits seuils minimum et maximum et en
ce que l'on répartit lesdites réponses en trois classes différentes, selon qu'elles sont supérieures au
seuil maximum, inférieures au seuil minimum ou comprises
entre ces deux seuils.

7 - Dispositif pour la mise en oeuvre du procédé
spécifié dans l'une quelconque des revendications
précédentes,
caractérisé en ce qu'il comporte, à poste fixe, d'une
part une source de lumière (12) et d'autre part un
système optique (14) et un agencement (15) de dispositifs photosensibles associés à des moyens de mémorisation des signaux qui en sont issus, des moyens mobiles
de support (1,2) pour faire défiler ledit récipient
entre ladite source de lumière et ledit système optique,
des moyens de commande (19) desdits dispositifs photosensibles, des moyens moteurs (3,5) pour mouvoir lesdits
moyens mobiles de support (1,2), des moyens de commande
(16) desdits moyens moteurs, des moyens de synchronisation (16) entre lesdits moyens de commande (16) des
dispositifs photosensibles (15) et lesdits moyens de
commande (16) des moyens moteurs (3,5) et des moyens
de traitement (16) des signaux provenant desdits moyens
de mémorisation.

4

0046430

8 - Dispositif selon la revendication 7,
caractérisé en ce que les moyens de commande des
dispositifs photosensibles, les moyens de commande des
moyens moteurs, lesdits moyens de synchronisation
et lesdits moyens de traitement sont rassemblés dans
un microprocesseur (16).

9 - Application du procédé spécifié selon l'une
des revendications 1 à 6 ou du dispositif selon l'une
des revendications 7 ou 8 à la reconnaissance de
caractères immunocytologiques.

Fig.1

Fig. 2

Fig. 3

0046430

Fig.4

Fig.5

Fig.6

Fig. 7

Fig. 8

## DOCUMENTS CONSIDERES COMME PERTINENTS

| Catégorie | Citation du document avec indication, en cas de besoin, des parties pertinentes | Revendication concernée |
|---|---|---|
| D | FR - A - 2 444 939 (AKRO-MEDIC ENGINEERING)  * pages 14-16; figures 8-11 * | 1,4,7, 9 |
| D | GB - A - 2 014 300 (OPTO ELEC-TRONIC DISPLAYS)  * page 1 * | 1,2 |
| A | EP - A - 0 012 698 (MERCK & CO.)  * pages 2,3 * | 1 |
| A | CLINICAL CHEMISTRY, vol. 25, no. 4, avril 1979 EASTON, PENN. (US) C.H. McMURRAY et al.: "Multi-Channel, Probe Colorimeter for Use with the Micro-ELISA Test, Which Makes Use of Disposable Flat-Bottom Microhemagglutina-tion Plates", pages 570-576.  * pages 570-572 * | 1,2 |
| A | US - A - 3 773 426 (C. MUDD)  * colonne 3; colonne 4; figures 1,4 * | 1 |

**CLASSEMENT DE LA DEMANDE (Int. Cl.³)**

G 01 N 21/25
G 01 N 33/48

**DOMAINES TECHNIQUES RECHERCHES (Int. Cl.³)**

G 01 N 21/25
G 01 N 33/48

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 23.11.1981 | BOEHM |

OEB Form 1503.1   06.78